(19) Europäisches Patentamt
European Patent Office
Office européen des brevets

(11) Veröffentlichungsnummer: **0 081 455**
**B1**

(12) # EUROPÄISCHE PATENTSCHRIFT

(45) Veröffentlichungstag der Patentschrift: **09.04.86**

(51) Int. Cl.[4]: **C 07 C 177/00**, A 61 K 31/557

(21) Anmeldenummer: **82730142.5**

(22) Anmeldetag: **03.12.82**

(54) **9-Brom-prostaglandinderivate, Verfahren zu ihrer Herstellung und ihre Verwendung als Arzneimittel.**

(30) Priorität: **04.12.81 DE 3148743**

(43) Veröffentlichungstag der Anmeldung: **15.06.83 Patentblatt 83/24**

(45) Bekanntmachung des Hinweises auf die Patenterteilung: **09.04.86 Patentblatt 86/15**

(84) Benannte Vertragsstaaten: **AT BE CH DE FR GB IT LI LU NL SE**

(56) Entgegenhaltungen:
**EP-A-0 030 377**
**US-A-4 156 087**

**CHEMICAL ABSTRACTS, Band 93, Nr. 5, 4. August 1980, Seite 866, Nr. 46010b, Columbus, Ohio, USA S. OHUCHIDA et al.: "Syntheses and biological activities of some prostacyclin analogs"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Schwarz, Norbert, Dr., Hauptstrasse 118, D-1000 Berlin 62 (DE)**
Erfinder: **Vorbrüggen, Helmut, Prof., Wilkestrasse 7, D-1000 Berlin 27 (DE)**
Erfinder: **Elger, Walter, Dr., Schorlemer Allee 12b, D-1000 Berlin 33 (DE)**

**EP 0 081 455 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue 9-Brom-prostaglandin-derivate, Verfahren zu ihrer Herstellung sowie ihre Verwendung als Arzneimittel.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

9-Desoxy-9-chlor-prostaglandinderivate sind in der Europäischen Patentanmeldung EP-A-0030377 beschrieben. In der US-Patentschrift US-A-4 156 087 wird als Zwischenprodukt zur Synthese einer 9-Desoxy-Verbindung der entsprechende 9-Desoxy-9-brom-16,16-dimethyl-$PGF_{2\alpha}$ -methylester erwähnt; biologische Daten dieser Verbindung werden nicht beschrieben.

Es wurde nun gefunden, daß die neuen 9-Bromprostaglandin-derivate nicht nur eine hervorragende Wirkungsspezifität, bessere Wirksamkeit, längere Wirkungsdauer als die natürlichen Prostaglandine besitzen; hervorzuheben ist vor allem auch eine größere Wirkungsspezifität gegenüber den 9-Chlor-prostaglandinderivaten.

Sie sind besonders für die orale Applikation geeignet.

Die Erfindung betrifft 9-Brom-prostanderivate der allgemeinen Formel I

Formel I

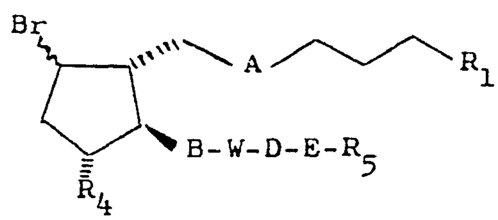

(I),

worin das 9-Bromatom α- oder β-ständig sein kann,

$R_1$ den Rest $CH_2OH$ oder

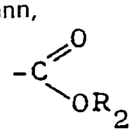

mit $R_2$ in der Bedeutung eines Wasserstoffatoms; einer $C_{1-10}$-Alkylgruppe; einer $C_{1-10}$-Alkylgruppe die substituiert ist durch einen Fluor-, Chlor-, Brom-, Phenyl-, Dimethylamino-, Diäthylamino-, Methoxy-, Äthoxy- oder einen durch den Chlor-, Brom-, Fluor-, Phenyl-, Methoxy- oder Äthoxy-Rest substituierten Phenyl-Rest; einer $C_{3-10}$-Cycloalkyl-gruppe; einer durch einen $C_{1-4}$-Alkyl-Rest substituierten $C_{3-10}$-Cycloalkylgruppe; einer Phenyl- oder Naphthylgruppe; einer Phenyl- oder Naphthylgruppe, die substituiert ist durch einen Phenyl-, Chlormethyl-, Fluormethyl-, Trifluormethyl-, Carboxyl-, Hydroxy-Rest, 1-3 Halogen-Atome, 1-3 $C_1$-$C_4$-Alkylgruppen oder einer $C_{1-4}$-Alkoxygruppe; eines 5-oder 6-gliedrigen Heterocyclus mit wenigstens einem Stickstoff-, Sauerstoff- oder Schwefel-Atom;

oder $R_1$ den Rest

$$-\overset{\overset{O}{\|}}{C}-NHR_3 \text{ mit}$$

$R_3$ in der Bedeutung eines Säurerestes oder des Restes $R_2$,

A eine $-CH_2-CH_2-$ oder cis-CH$=$CH-Gruppe,

B eine $-CH_2-CH_2-$, oder trans-CH$=$CH- oder eine -C$\equiv$C-Gruppe,

W eine freie oder funktionell abgewandelte Hydroxymethylengruppe oder eine freie oder funktionell abgewandelte

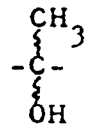

Gruppe, wobei die OH-Gruppe α- oder β-ständig sein kann,

D und E gemeinsam eine direkte Bindung oder

D eine geradkettige, verzweigtkettige oder ringförmige Alkylengruppe mit bis zu 10 C-Atomen, die gegebenenfalls durch Fluoratome substituiert sein kann, und gegebenenfalls eine Doppelbindung in 2,3-oder 4-Stellung enthält,

E ein Sauerstoffatom oder Schwefelatom oder eine direkte Bindung oder eine $-C{\equiv}C$-Gruppe oder eine $-CR_6{=}CR_7$-Gruppe darstellt, wobei $R_6$ und $R_7$ sich unterscheiden und ein Wasserstoffatom oder ein Chloratom oder eine $C_1$-$C_4$-Alkyl-gruppe bedeuten,

$R_4$ eine freie oder funktionell abgewandelte Hydroxygruppe,

$R_5$ eine Alkyl- oder Alkenylgruppe mit bis zu 10 C-Atomen; eine Alkinylgruppe mit bis zu 6 C-Atomen; eine Alkinylgruppe mit bis zu 6 C-Atomen, die in 1-Stellung substituiert ist durch Fluor oder einen $C_{1-4}$-Alkylrest; eine $C_{3-10}$-Cycloalkylgruppe; eine durch einen $C_{1-4}$-Alkyl-Rest substituierte $C_{3-10}$-Cycloalkylgruppe; eine Phenyl- oder Naphtylgruppe; eine Phenyl- oder Naphthylgruppe, die substituiert ist durch einen Phenyl-, Chlormethyl-, Fluormethyl-, Trifluormethyl-, Carboxyl-, Hydroxy-Rest, 1-3 Halogenatome, 1-3 $C_{1-4}$-Alkylgruppen oder eine $C_{1-4}$-Alkoxygruppe; einen 5- oder 6-gliedrigen Heterocyclus mit wenigstens einem Stickstoff-, Sauerstoff- oder Schwefelatom; und falls $R_2$ die Bedeutung eines Wasserstoffatoms hat, deren Salze mit physiologisch verträglichen Basen bedeuten, mit der Maßgabe, daß die Gruppierung D-E-$R_5$ nicht den Rest 1,1-Dimethylpentyl darstellt, wenn A für cis-$CH{=}CH$, B für trans-$CH{=}CH$-, W für CHOH, $R_4$ für OH, $R_1$ für $COOCH_3$ stehen und der 9-Brom-Substituent β-ständig ist.

Als Alkylgruppen $R_2$ sind gerade oder verzweigte Alkylgruppen mit 1-10 C-Atomen zu betrachten, wie beispielsweise Methyl, Äthyl, Propyl, Butyl, Isobutyl, tert.-Butyl, Pentyl, Neopentyl, Hexyl, Heptyl, Decyl. Die Alkylgruppen $R_2$ können gegebenenfalls ein- bis mehrfach substituiert sein durch Halogenatome, Alkoxygruppen, gegebenenfalls substituierte Aryl- bzw. Aroylgruppen, Dialkylamino und Trialkylammonium, wobei die einfache Substitution bevorzugt sein soll. Als Substituenten seien beispielsweise genannt Fluor, Chlor oder Brom, Phenyl, Dimethylamino, Diäthylamino, Methoxy, Athoxy. Als bevorzugte Alkylgruppen $R_2$ sind solche mit 1-4 C-Atomen, wie z.B. Methyl, Äthyl, Propyl, Dimethylaminopropyl, Isobutyl, Butyl zu nennen.

Als Arylgruppen $R_2$ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe $R_2$ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl und Adamantyl.

Als heterocyclische Gruppen $R_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest $R_3$ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclcpentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Alkansulfonsäuren mit 1-10 C-Atomen wie z.B. Methansulfonsäure, Äthansulfonsäure, Isopropansulfonsäure und Butansulfonsäure sowie β-Chloräthansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Die Hydroxygruppen in W und $R_4$ können funktionell abgewandelt sein, beispielsweise durch Verätherung oder Veresterung, wobei auch die abgewandelte Hydroxygruppe in W α- oder β-ständig sein kann, wobei freie hydroxygruppen bevorzugt sind.

Als Äther- und Acylreste kommen die dem Fachmann bekannten Reste in Betracht. Bevorzugt sind leicht abspaltbare Ätherreste, wie beispielsweise der Tetrahydropyranyl-, Tetrahydrofuranyl-, α-Äthoxyäthyl-, Trimethylsilyl-, Dimethyl-tert.-butyl-silyl- und Tribenzyl-silylrest. Als Acylreste kommen die gleichen wie für $R_3$ unter organischen Carbonsäuren genannt in Frage, namentlich genannt seien beispielsweise Acetyl, Propionyl, Butyryl und Benzoyl Als Alkylgruppen $R_5$ kommen gerad- und verzweigtkettige Alkyl- und Alkenylreste mit bis zu 10, insbesondere bis zu 6 C-Atomen, in Frage, die gegebenenfalls durch gegebenenfalls substituiertes Phenyl, Alkyl mit 1-4 C-Atomen oder Halogen substituiert sein können, und für den Fall, daß D und E gemeinsam eine Direktverbindung bedeuten, gegebenenfalls in 1-Stellung durch Fluor oder $C_1$-$C_4$-Alkyl substituiertes Alkinyl mit bis zu 6 C-Atomen. Beispielsweise genannt seien Methyl, Äthyl, Propyl, Isobutyl, tert.-Butyl, Pentyl, Hexyl, Heptyl, Octyl, Butenyl, Isobutenyl, Propenyl, Pentenyl, Hexenyl und Benzyl. Als Alkinylreste kommen in Betracht: Äthinyl, Propin-1-yl, Propin-2-yl, 1-Methylpropin-2-yl, 1-Fluorpropin-2-yl, 1-Äthylpropin-2-yl, 1-Fluorbutin-2-yl, Butin-2-yl, Butin-3-yl, 1-Methyl-butin-3-yl, 1-Methylpentin-3-yl, 1-Fluor-pentin-3-yl, 1-Methyl-pentin-2-yl, 1-Fluorpentin-2-yl, 1-Methylpentin-4-yl, 1-Fluorpentin-4-yl, Hexin-1-yl, 1-Methylhexin-2-yl, 1-Fluor-hexin-2-yl, 1-Methylhexin-3-yl, 1-Methylhexin-4-yl, Hexin-3-yl, 1,1-Dimethylpropin-2-yl, 1,1-Dimethylbutin-3-yl, 1,1-Dimethyl-pentin-3-yl, 1,1-Dimethylpentin-4-yl, 1,1-Dimethylhexin-3-yl, 1,1-Dimethylhexin-4-yl usw.. Für Halogen als Substituent der Alkyl- und Alkenylgruppen $R_5$ kommen Brom, Chlor und Fluor in Betracht. Bevorzugt sind Chlor und Fluor.

Die Cycloalkylgruppe $R_5$ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seien Cyclopropyl, Cyclobutyl, Cyclopentyl, Cyclohexyl, Methyl-cyclohexyl und Adamantyl.

Als substituierte bzw. unsubstituierte Arylgruppen $R_5$ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische Gruppen $R_5$ kommen 5- und 6-gliedrige ge Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe D kommen geradkettige oder verzweigtkettige, ringförmige, gesättigte und ungesättigte Alkylenreste, vorzugsweise gesättigte mit bis zu 10, insbesondere bis zu 5 C-Atomen, in Frage, die gegebenenfalls durch Fluoratome substituiert sein können. Beispielsweise seien genannt: Methylen, Fluormethylen, Difluormethylen, Äthylen, 1,2-Propylen, Äthyläthylen, Trimethylen, Tetramethylen, Pentamethylen, 1,1-Difluoräthylen, 1-Fluoräthylen, 1-Methyl-tetramethylen, 1-Methyl-tri-methylen, 1-Methylen-äthylen, 1-Methylen-tetramethylen, 2-Methyl-trimethylen, 2-Methyl-tetramethylen, 1,1-Trimethylen-äthylen, 1,2-Methylenäthylen. Wenn eine Doppelbindung vorliegt, befindet sie sich in den Alkylenresten in 2-, 3- oder 4-Stellung.

Geeignete Alkylgruppen $R_6$ und $R_7$ schließen Gruppen mit 1-4 C-Atomen ein, wie sie für $R_2$ bereits erwähnt wurden.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ausserdem ein Verfahren zur Herstellung der erfindungsgemässen 9-Brom-prostanderivate der allgemeinen Formel I, dadurch gekennzeichnet, dass man in an sich bekannter Weise eine Verbindung der allgemeinen Formel II,

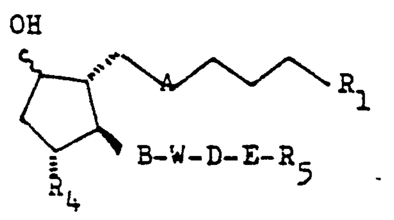

(II),

worin die CH-Gruppe α- oder β-ständig sein kann $R_1$ die Reste

$$-\overset{O}{\overset{\|}{C}}-OR_2 \text{ oder } -\overset{O}{\overset{\|}{C}}-NHR_3$$

(mit $R_2$ außer Wasserstoff und $R_3$ in den bereits angegebenen Bedeutungen) darstellt und A, B, D, E und $R_5$ die obenangegebenen Bedeutungen haben und freie OH-Gruppen in $R_4$ und W geschützt sind, mit dem Reagenz Tetrabrommethan/Triphenylphosphin bromiert und gegebenenfalls anschließend in beliebiger Reihenfolge geschützte Hydroxygruppen freisetzt und/oder freie Hydroxygruppe verestert, veräthert und/oder Doppelbindungen hydriert und/oder eine veresterte Carboxylgruppe ($R_1$ =

$$-\overset{O}{\overset{\|}{C}}-OR_2)$$

verseift und/oder eine freie Carboxylgruppe ($R_2$=H) in ein Amid ($R_1$ =

$$-\overset{O}{\overset{\|}{C}}-NHR_3)$$

überführt und/oder eine freie oder veresterte Carboxylgruppe ($R_1$ =

$$-\overset{O}{\overset{\|}{C}}-OR_2)$$

reduziert.

Die Umsetzung der Verbindungen der allgemeinen Formel II zu den Verbindungen der allgemeinen Formel I mit Tetrabrommethan und Triphenylphosphin erfolgt in einem inerten lösungsmittel wie beispielsweise Dimethylformamid, Dimethylacetamid, Acetonitril, Methylenchlorid bei Temperaturen zwischen 0°C und 80°C, vorzugsweise 2C°C bis 45°C in Gegenwart einer Base wie beispielsweise Pyridin, Triäthylamin usw.

Setzt man einen Alkohol der allgemeinen Formel II mit einer β-ständigen 9-Hydroxygruppe ein, so erhält man Verbindungen der allgemeinen Formel I mit 9-α-ständigem Bromatom, setzt man einen Alkohol mit einer α-ständigen Hydroxygruppe ein, so erhält man Verbindungen mit 9-β-ständigem Bromatom.

Die Reduktion zu den Verbindungen der allgemeinen Formel I mit $R_1$ in der Bedeutung einer -$CH_2OH$-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diäthyläther, Tetrahydrofuran, Dimethoxyäthan, Toluol usw. in Frage. Die Reduktion wird bei Temperaturen von 30°C bis zur Siedetemperatur des verwendeten Lösungsmittels, vorzugsweise 0°C bis 30°C vorgenommen.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z.B. Oxhlsäure, Essigsäure, Propionsäure u.a., oder in einer wässrigen Lösung einer anorganischen Säure, wie z.B. Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind z.B. Alkohole wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohole kommen aliphatische Alkohole in Betracht, wie z.B. Methanol, Äthanol, Butanol usw., vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und

Bariumcarbonat. Die Umsetzung erfolgt bei -10°C bis + 70°C, vorzugsweise bei + 25°C.

Die Einführung der Estergruppe

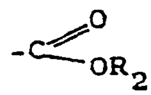

für $R_1$, bei welcher $R_2$ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z.B. dadurch, dass man eine Lösung des Diazokohlenwasserstoffes in einem inerten Lösungsmittel vorzugsweise in Diäthyläther, mit der 1-Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nhch bekannten Methoden hergestellt werden [Org.Reactions Bd. 8, Seiten 389-394 (1954)]. Die Einführung der Estergruppe

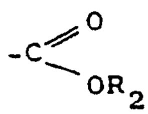

für $R_1$, bei welcher $R_2$ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base beispielsweise Pyridin Dimethylaminopyridin, Triäthylamin, in inerten Lösungsmitteln umgesetzt. Als Lösungsmittel kommen Methylenchlorid, Äthylenchlorid, Chloroform, Essigester, Tetrahydrofuran, vorzugsweise Chloroform in Frage. Die Reaktion wird bei Temperaturen zwischen -30°C und + 50°C, vorzugsweise bei 10°C, durchgeführt.

Sollen im Primärprodukt enthaltene C=C-Doppelbindungen reduziert werden, erfolgt die Hydrierung nach an sich bekannten Methoden.

Die Hydrierung der 5,6-Doppelbindung wird in an sich bekannter Weise bei tiefen Temperaturen, vorzugsweise bei etwa -20°C, in einer Wasserstoffatmosphäre in Gegenwart eines Edelmetallkatalysators durchgeführt. Als Katalysator ist zum Beispiel 10% Palladium auf Kohle geeignet.

Werden sowohl die 5,6- als auch die 13,14-Doppelbindung hydriert, so arbeitet man bei höherer Temperatur vorzugsweise bei etwa 20°C.

Die Prostaglandinderivate der allgemeinen Formel I mit $R_2$ in der Bedeutung eines Wasserstoffatoms können mit geeigneten Mengen der entsprechenden anorganischen Basen unter Neutralisierung in ein Salz überführt werden. Beispielweise erhält man beim Lösen der entsprechenden PG— säuren in Wasser, das die stöchiometrische Menge der Base enthält nach Abdampfen des Wassers oder nach Zugabe eines mit Wasser mischbaren Lösungsmittels, z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Äthanol, Aceton, Diäthyläther, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe

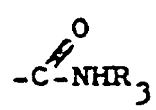

für $R_1$ erfolgt nach den dem Fachmann bekannten Methoden. Die Carbonsäuren der allgemeinen Formel I ($R_2$=H), werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$=H) oder des entsprechenden Amins erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen —30°C und +60°C, vorzugsweise bei 0°C bis 30°C.

Eine weitere Möglichkeit für die Einführung der Amidgruppe

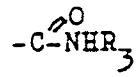

für $R_1$ mit $R_3$ in der Bedeutung eines Säurerestes besteht in der Umsetzung einer 1-Carbonsäure der allgemeinen Formel I ($R_2=H$), in der freie Hydroxygruppen gegebenenfalls intermediär geschützt sind, mit Verbindungen der allgemeinen Formel III,

$O = C = N - R_3$ (III),

worin $R_3$ die obenangegebene Bedeutung hat.

Die Umsetzung der Verbindung der allgemeinen Formel I ($R_2=H$) mit einem Isocyanat der allgemeinen Formel III erfolgt gegebenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen -80°C bis 100°C, vorzugsweise bei 0 bis 30°C,vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II können beispielsweise hergestellt werden, in dem man in an sich bekannter Weise ein Keton der allgemeinen Formel IV

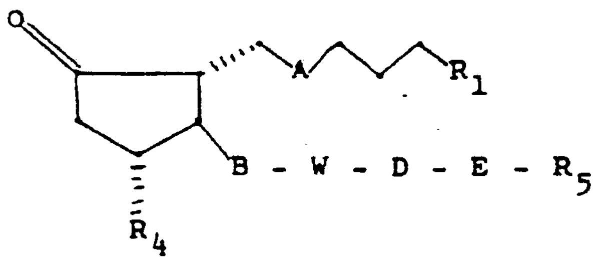

(IV)

worin $R_1$ die Reste

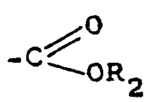 oder 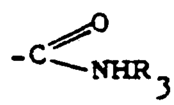

bedeutet und A,B,D,E und $R_5$ die obenangegebenen Bedeutungen haben und freie OH-Gruppen in $R_4$ und W geschützt sind, mit Natriumborhydrid, Lithium-tris(tert.-butoxy)aluminiumhydrid usw. reduziert und gebenenfalls anschliessend die epimeren 9α- und 9β-ständigen Hydroxyverbindungen der allgemeinen Formel II trennt.

Im Vergleich zu PGE-Derivaten zeichnen sich die neuen 9-Brom -prostaglandine durch grössere Stabilität aus.

Die neuen 9-Brom -prostanderivate der allgemeinen Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die neuen Prostaglandin-Analoga wirken stark luteolytisch, d.h. zur Auslösung einer Luteolyse benötigt man wesentlich geringere Dosierungen als bei den entsprechenden natürlichen Prostaglandinen.

Auch zur Auslösung von Aborten, insbesondere nach oraler oder intravaginaler Applikation, sind geringere Mengen der neuan Prostaglandinanaloga im Vergleich zu den natürlichen Prostaglandinen erforderlich.

Bei der Registrierung der isotonischen Unteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemässen Prostaglandin-Derivate zur Cervixdilatation als Vorbereitung für diagnostische oder therapeutische Eingriffe.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemässen Substanzen wirken auch bronchospasmolytisch. Ausserdem bewirken sie eine Abschwellung der Nasenschleimhaut.

Die erfindungsgemässen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen. Sie wirken ausserdem an der Leber und auch an der Bauchspeicheldrüse zytoprotektiv.

Einige der Verbindungen wirken blutdrucksenkend, regulierend bei Herzrhythmusstörungen und hemmend auf die Plättchenaggregation mit den sich daraus ergebenden Einsatzmöglichkeiten. Die neuen Prostaglandine können auch in Kombination z.B. mit D-Blockern und Diuretika verwendet werden.

Die erfindungsgemäßen 9-Bromprostaglandine schützen die Magenschleimhaut von Ratten bei oraler Gabe noch bei einer Dosis von $EG_{50}$=0,01-0,1 Mikrogramm pro Kilogramm vor der ulcerogenen Wirkung von Indomethacin und von anderen Noxen. Die abortive Wirkung der Verbindungen ist relativ gering und entspricht etwa der von Prostaglandin $E_2$.

Die Dosis der Verbindungen ist 1-1500 µg/kg/Tag, wenn sie am menschlichen Patienten verabreicht werden.

Für die medizinische Anwendung können die Wirkstoffe in eine für die Inhalation, für orale, parenterale oder lokale (z.B. vaginale) Applikation geeignete Form überführt werden.

Zur Inhalation werden zweckmässigerweise Ärosollösungen hergestellt.

Für die orale Applikation sind beispielsweise Tabletten, Dragees oder Kapseln geeignet.

Für die parenterale Verabreichung werden sterile, injizierbare, wässrige oder ölige Lösungen benutzt.

Für die vaginale Applikation sind z.B. Zäpfchen geeignet und üblich.

Die Erfindung betrifft damit auch Arzneimittel auf Basis der Verbindungen der allgemeinen Formel I und der üblichen Hilfs- und TRägerstoffe.

Die erfindungsgemässen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt oder zur Behandlung der Hypertonie dienen. Für diesen Zweck aber auch für die übrigen Anwendungen können die Präparate 0,01 - 100 mg der aktiven Verbindung enthalten.

Die folgenden Beispiele sollen die Erfindung näher erläutern, ohne dass damit eine Begrenzung vorgenommen wird.

**Beispiel 1**

**(5Z,13E)-(9R,11R,15R)-9-Brom-11,15-dihydroxy-16– phenoxy-17,18,19,20-tetranor-5,13,prostadiensäuremethylester**

Eine Lösung aus 1,15 g (5Z,13E)-(9S,11R,15R)-9-Hydroxy-11,15-bis-(tetrahydropyran-2-yloxy)-16-phenoxy-17,18,19,20-tetranor-5,13-prostadiensäuremethylester, 772 mg Triphenylphosphin, 0,3 g Pyridin und 1,25 g Tetrabrommethan in 18 ml Acetonitril rührt man 27 Stunden bie 22 °C. Anschließend verdünnt man mit 50 ml Wasser, extrahiert dreimal mit einer Mischung aus Äther/Hexan (1+1), wäscht den organischen Extrakt mit Wasser, trocknet über Magnesiumsulfat und dampft im Vakuum ein. Nach Chromatographie des Eindampfrückstands an Kieselgel erhält man mit Hexan/Äther (4+1) 690 mg (5Z,13E)-(9R,11R,15R)-9-Brom-11,15-bis-(tetra-hydropyran-2-yloxy)-16-phenoxy-17,18,19,20-tetranor-5,13-prostadiensäure-methylester.

IR ($CHCl_3$): 2940, 2856, 1731, 1600, 1588, 1495, 972/cm.

Zur Tetrahydropyranyläther-Abspaltung rührt man 690 mg des vorstehend erhaltenen Produktes 16 Stunden bei 22 °C mit 30 ml einer Mischung aus Essigsäure/Wasser/Tetrahydrofuran (65+35+lO) und dampft anschließend im Vakuum ein. Den Rückstand reinigt man durch Chromatographie an Kieselgel. Mit Toluol/Isopropanol (98+2) erhält man 225 mg der Titelverbindung als farbloses Öl.

IR: 3600, 3420 (breit), 2930, 2857, 1730, 1599, 1494, 970/cm.

**Beispiel 2**

**(5Z,13E)-(9S,11R,15R)-9-Brom-11,15-dihydroxy-16– phenoxy-17,18,19,20-tetranor-5,13-prostadiensäuremethylester**

Zu einer Lösung aus 574 mg (5Z,13E)-(9R,11R,15R)-9-Hydroxy-11,15-bis-(tetrahydropyran-2-yloxy)-16-phenoxy-17,18,19,20-tetranor-5,13-prostadien-säure-methylester, 0,52 g Tetrabrommethan und 0,125 g Pyridin in 7,5 ml Acetonitril fügt man 386 mg Triphenylphosphin und rührt 24 Stunden bei 22 °C. Anschließend verdünnt man mit Wasser, extrahiert dreimal mit einer Mischung aus Ather/Hexan (1+1), wäscht den organischen Extrakt mit Wasser, trocknet über Magnesiumsulfat und dampft im Vakuum ein. Nach Chromatographie des

Eindampfrückstands an Kieselgel erhält man mit Hexan/Äther (7+3) 380 mg (5Z,13E)-(9S,11R,15R)-9-Brom-11,15-bis-(tetra-hydropyran-2-yloxy)-16-phenoxy-17,18,19,20-tetranor-5,13-prostadiensäure-methylester.
IR: 2940, 2857, 1730, 1600, 1588, 1494, 970/cm.

Zur Tetrahydropyranyläther-Abspaltung rührt man 380 mg der vorstehend erhaltenen 9α-Bromverbindung 16 Stunden bei 22 °C mit 18 ml einer Lösung aus Essigsäure/Wasser/-Tetrahydrofuran (65+35+10) und dampft anschließend im Vakuum ein. Den Rückstand reinigt man durch Chromatographie an Kieselgel. Mit Essigester/Hexan (7+3) erhält man 220 mg der Titelverbindung als Öl.
IR: 3590, 3420 (breit), 2930, 2860, 1730, 1600, 1589, 1495, 971/cm.

**Beispiel 3**

**(13E)-(9R,11R,15R)-9-Brom-11,15-dihydroxy-16-phenoxy— 17,18,19,20-tetranor-13-prostensäure-methylester**

In Analogie zu Beispiel 1 erhält man aus 0,65 g (13E)-(9S,11R,15R)-9-Hydroxy-11,15-bis-(tetrahydropyran-2-yloxy)-16-phenoxy-17,18,19,20-tetranor-13-prosten-säuremethylester 310 mg (13E)-(9R,11R,15R)-9-Brom-11,15-bis-(tetrahydropyran-2-yloxy)-16-phenoxy-17,18,-19,20-tetranor-13-prostensäuremethylester.
IR: 2942, 2857, 1731, 1600, 1588, 971/cm.

Nach Abspaltung der Schutzgruppen gemäß Beispiel 1 erhält man 98 mg der Titelverbindung als farbloses Öl.
IR: 3610, 3410 (breit), 2931, 2858, 1731, 1599, 1588, 971/cm.

**Beispiel 4**

**(13E)-(9S,11R,15R)-9-Brom-11,15-dihydroxy-16-phenoxy— 17,18,19;20-tetranor-13-prostensäure-methylester**

In Analogie zu Beispiel 2 erhält man aus 300 mg (13E)-(9R,11R,15R)-9-Hydroxy-11,15-bis-(tetrahydropyran-2-yloxy)-16-phenoxy-17,18,19,20-tetranor— 13-prostensäuremethylester 195 mg (13E)-(9S,11R,15R)-9-Brom-11,15-bis-(tetrahydropyran-2-yloxy)-16-phenoxy-17,18,19,20-tetranor-13-prostensäuremethylester.
IR: 2940, 2858, 1730, 1600, 1588, 1494, 970/cm.

Nach Abspaltung der Schutzgruppen gemäß Beispiel 2 erhalt man 110 mg der Titelverbindung als farbloses Öl.
IR: 3600, 3400 (breit), 2930, 2858, 1730, 1599, 1589, 970/cm.

**Beispiel 5**

**(5Z,13E)-(9R,11R,15R)-9-Brom-11,15-dihydroxy-16,16— dimethyl-5,13-prostadiensäuremethylester**

In Analogie zu Beispiel 1 erhält man aus 0,7 g (5Z,13E)-(9S,11R,15R)-16,16-Dimethyl-9-hydroxy-11,15-bis-(tetrahydropyran-2-yloxy)-5,13-prostadiensäuremethylester 0,38 g (5Z,13E)-(9R,11R,15R)-9-Brom-11,15-bis-(tetrahydropyran-2-yloxy)-16,16-dimethyl-5,13-prostadiensäuremethylester.
IR: 2942, 2858, 1732, 972/cm.

Nach Abspaltung der Schutzgruppen gemäß Beispiel 1 erhält man 120 mg der Titelverbindung als farbloses Öl.
IR: 3600, 3400 (breit), 2935, 1732, 973/cm.

**Beispiel 6**

**(5Z,13E)-(9S,11R,15R)-9-Brom-11,15-dihydroxy-16,16— dimethyl-5,13-prostadiensäuremethylester**

In Analogie zu Beispiel 2 erhält man aus 190 mg (5Z,13E)-(9R,11R,15R)-16,16-Dimethyl-9-hydroxy-11,15-bis-(tetra-hydropyran-2-yloxy)-5,l3,prostadiensäuremethylester 108 mg (5Z,13E)-(9S,11R,15R)-9-Brom-16,16-dimethyl-11,15-bis-(tetrahydropyran-2-yloxy)-5,13-prostadiensäuremethylester.
IR: 2943, 2860, 1733, 973/cm.

Nach Abspaltung der Schutzgruppen gemäß Beispiel 2 erhalt man 65 mg der Titelverbindung als farbloses Öl.
IR: 3610, 3410 (breit), 2935, 1733, 973/cm.

**Beispiel 7**

**(5Z,13E)-(9R,11R,15S,16RS)-9-Brom-11,15-dihydroxy— 16-methyl-5,13-prostadiensäuremethylester**

In Analogie zu Beispiel 1 erhält man aus 0,9 g (5Z,13E)-(9S,11R,15S,16RS)-9-Hydroxy-16-methyl-11,15-bis-(tetrahydropyran-2-yloxy)-5,13-prostadiensäuremethylester 0,43 g (5Z,13E)-(9R,11R,15S,16RS)-9-Brom-16-methyl-11,15-bis-(tetrahydropyran-2-yloxy)-5,13-prostadiensäuremethylester.
IR: 2940, 2858, 1731, 971/cm.
Nach Abspaltung der Schutzgruppen gemäß Beispiel 1 erhält man 138 mg der Titelverbindung als farbloses Öl.
IR: 3600, 3420 (breit), 2936, 2860, 1731, 971/cm.

**Beispiel 8**

**(5Z,13E)-(9S,11R,15S,16RS)-9-Brom-11,15-dihydroxy— 16-methyl-5,13-prostadiensäuremethylester**

In Analogie zu Beispiel 2 erhält man aus 280 mg (5Z, 13E)-(9R,11R,15S,16RS)-9-Hydroxy-16-methyl-11,15-bis-(tetrahydropyran-2-yloxy)-5,13-prostadiensäuremethylester 165 mg (5Z,13E)-(9S,11R,15S,16RS)-9-Brom-16-methyl-11,15-bis-(tetrahydropyran-2-yloxy)-5,13-prostadiensäuremethylester.
IR: 2940, 2860, 1732, 972/cm.
Nach Abspaltung der Schutzgruppen gemäß Beispiel 2 erhält man 90 mg der Titelverbindung als farbloses Öl.
IR: 3620, 3400 (breit), 2935, 2862, 1732, 972/cm.

**Beispiel 9**

**(5Z,13E)-(9R,11R,15RS)-9-Brom-11,15-dihydroxy-15— methyl-5,13-prostadiensäuremethylester**

In Analogie zu Beispiel 1 erhält man aus 1,2 g (5Z,13E)-(9S,11R,15RS)-9-Hydroxy-15-methyl-11,15-bis-(tetrahydropyran-2-yloxy)-5,13-prostadiensäure methylester 680 mg (5Z,13E)-(9R,11R,15RS)-9-Brom-15-methyl-11,15-bis-(tetrahydropyran-2-yloxy)-5,13-prostadiensäuremethylester.
IR: 1730, 974/cm.
Nach Abspaltung der Schutzgruppen gemäß Beispiel 1 erhält man 260 mg der Titelverbindung als farbloses Öl.
IR: 3620, 3420 (breit), 2938, 1730, 974/cm.

**Beispiel 10**

**(13E)-(9R,11R,15S,16RS)-9-Brom-11,15-dihydroxy-16,19— dimethyl-13,18-prostadiensäuremethylester**

In Analogie zu Beispiel 1 erhält man aus 0,8 g (13E)-(9S,11R,15S,16RS)-16,19-dimethyl-9-hydroxy-11,15-bis-(tetrahydropyran-2-yloxy)-13,18-prostadiensäuremethylester 480 mg (13E)-(9R,11R,15S,16RS)-9-Brom-16,19-dimethyl-11,15-bis-(tetrahydropyran-2-yloxy)-13,18-prostadiensäuremethylester.
IR: 2962, 1731, 972/cm.
Nach Abspaltung der Schutzgruppen gemäß Beispiel 1 erhält man 220 mg der Titelverbindung als farbloses Öl.
IR: 3610, 3410 (breit), 2933, 2856, 1731, 972/cm.

**Beispiel 11**

**(13E)-(9S,11R,15S,16RS)-9-Brom-11,15-dihydroxy-16,19— dimethyl-13,13-prostadiensäuremethylester**

In Analogie zu Beispiel 2 erhält man aus 0,38 g (13E)-(9R,11R,15S,16RS)-16,19-dimethyl-9-hydroxy-11,15-bis-(tetrahydropyran-2-yloxy)-13,18-prostadiensäure methylester 240 mg (13E)-(9S,11R,15S,16RS)-9-Brom-16,19-dimethyl-11,15-bis-(tetrahydropyran-2-yloxy)-13,18-prostadiensäuremethylester.
IR: 2960, 1730, 970/cm.
Nach Abspaltung der Schutzgruppen gemäß Beispiel 2 erhält man 160 mg der Titelverbindung als farbloses Öl.

IR: 3600, 3400 (breit), 2935, 2855, 1730, 970/cm.

**Beispiel 12**

**(5Z,13E)-(9R,11R,15R)-9-Brom-11,15-dihydroxy-16,16,19– trimethyl-5,13,18-prostatriensäuremethylester**

In Analogie zu Beispiel 1 erhält man aus 0,65 g (5Z, 13E)-(9S,11R,15R)-9-Hydroxy-11,15-bis-(tetrahydropyran-2-yloxy)-16,16,19-trimethyl-5,13,18-prostatriensäuremethylester 0,38 g (5Z,13E)-(9R,11R,15R)-9-Brom-11,15-bis-(tetrahydropyran-2-yloxy)-16,16,19-trimethyl-5,13,18-prostatriensäuremethylester.
IR: 2962, 1732, 970/cm.
Nach Abspaltung der Schutzgruppen gemäß Beispiel 1 erhält man 0,18 g der Titelverbindung als farbloses Öl.
IR: 3600, 3400 (breit), 2942, 2850, 1732, 970/cm.

**Bespiel 13**

**(5Z,13E)-(9S,11R,15R)-9-Brom-11,15-dihydroxy-16,16,19– trimethyl-5,13,18-prostatriensäuremethylester**

In Analogie zu Beispiel 2 erhält man aus 0,7 g (5Z, 13E)-(9R,11R,15R)-9-Hydroxy-11,15-bis-(tetrahydro-pyran-2-yloxy)-16,16,19-trimethyl-5,13,18-prostatriensäuremethylester 0,48 g (5Z,13E)-(9S,11R,15R)-9-Brom-11,15-bis-(tetrahydropyran-2-yloxy)-16,16,19-trimethyl-5,13,18-prostatriensäuremethylester.
IR: 2960, 1733, 972/cm.
Nach Abspaltung der Schutzgruppen gemäß Beispiel 2 erhält man 0,36 g der Titelverbindung als farbloses Öl.
IR: 3600, 3410 (breit), 2941, 2853, 1733, 972/cm.

**Beispiel 14**

**(5Z,13E)-(9R,11R,15R)-9-Brom-11,15-dihydroxy-16-phenoxy– 17,18,19,20-tetranor-5,13-prostadiensäure**

225 mg des nach Beispiel 1 hergestellten Methylesters rührt man 1 Stunde mit 12 ml einer Lösung aus Kaliumhydroxid in Äthanol und Wasser (Herstellung: Man lost 2 g Kaliumhydroxid in 75 ml Äthanol und 25 ml Wasser). Anschließend sauert man mit 10 %iger Zitronensäurelösung auf pH 4 an, extrahiert dreimal mit Methylenchlorid, wascht den organischen Extrakt einmal mit Sole, trocknet über Magnesiumsulfat und dampft im Vakuum ein. Nach Chromatographie des Ruckstands an Kieselgel mit Essigester, Essigsäure (99,5 -0,5) als Elutionsmittel erhält man 168 mg der Titelverbindung als farbloses Öl.
IR: 3600, 3430 (breit), 2925, 2855, 1710, 1599, 1587, 1493, 970/cm

**Beispiel 15**

**(5Z,13E)-(9S,11R,15R)-9-Brom-11,15-dihydroxy-16– phenoxy-17,18,19,20-tetranor-5,13-prostadiensäure**

In Analogie zu Beispiel 14 erhält man aus 0,14 g des nach Beispiel 2 hergestellten Methylesters 115 mg der Titelverbindung als wachsartige Masse.
IR: 3590, 3400 (breit), 2930, 2853, 1710, 1599, 1588, 1492, 969/cm.

**Beispiel 16**

**(13E)-(9R,11R,I5R)-9-Brom-11,15-dihydroxy-16-phenoxy– 17,18,19,20-tetranor-13-prostensäure**

In Analogie zu Beispiel 14 erhält man aus 0,19 g des nach Beispiel 3 hergestellten Methylesters 0,14 g der Titelverbindung als farbloses Öl.
IR: 3600, 3400 (breit), 2932, 2853, 1709, 1599, 1588, 1492, 970/cm.

**Beispiel 17**

**(13E)-(9S,11R,15R)-9-Brom-11,15-dihydroxy-16-phenoxy– 17,18,19,20-tetranor-13-prostensäure**

In Analogie zu Beispiel 14 erhält man aus 0,3 g des nach Beispiel 4 hergestellten Methylesters 0,24 g der Titelverbindung als farbloses Öl.
IR: 3610, 3420 (breit), 2933, 2852, 1710, 1599, 1588, 1493, 971/cm.

**Beispiel 18**

**(5Z,13E)-(9R,11R,15R)-9-Brom-11,15-dihydroxy-16,16– dimethyl-5,13-prostadiensäure**

In Analogie zu Beispiel 14 erhält man aus 0,24 g des nach Beispiel 5 hergestellten Methylesters 0,18 g der Titelverbindung als farbloses 01.
IR: 3600, 3410 (breit), 2935, 1709, 973/cm.

**Beispiel 19**

**(5Z,13E)-(9S,11R,15R)-9-Brom-11,15-dihydroxy-16,16-dimethyl-5,13-prostadiensäure**

In Analogie zu Beispiel 14 erhält man aus 0,14 g des nach Beispiel 6 hergestellten Methylesters 0,10 g der Titelverbindung als farbloses Öl.
IR: 3600, 3410 (breit), 2938, 1710, 974/cm.

**Beispiel 20**

**(5Z,13E)-(9R,11R,15S,16RS)-9-Brom-11,15-dihydroxy– 16-methyl-5,13-prostadiensäure**

In Analogie zu Beispiel 14 erhält man aus 0,14 g des nach Beispiel 7 hergestellten Methylesters 0,11 g der Titelverbindung als farbloses Öl.
IR: 3600, 3405 (breit), 2940, 1710, 978/cm.

**Beispiel 21**

**(5Z,13E)-(9S,11R,15S,16RS)-9-Brom-11,15-dihydroxy– 16-methyl-5,13-prostadiensäure**

In Analogie zu Beispiel 14 erhält man aus 0,2 g des nach Beispiel 8 hergestellten Methylesters 0,16 g der Titelverbindung als farbloses Öl.
IR: 3600, 3405 (breit), 2938, 1710, 916/cm.

**Beispiel 22**

**(5Z,13E)-(9R,11R,15RS)-9-Brom-11,15-dihydroxy-15-methyl-5,13-prostadiensäure**

In Analogie zu Beispiel 14 erhält man aus 0,25 g des nach Beispiel 9 hergestellten Methylesters 0,20 g der Titelverbindung als farbloses Öl.
IR: 3600, 3410 (breit), 2945, 2860, 1712, 978/cm.

**Beispiel 23**

**(13E)-(9R,11R,15S,16RS)-9-Brom-11,15-dihydroxy-16,19-dimethyl-13,18-prostadiensäure**

In Analogie zu Beispiel 14 erhält man aus 0,25 g des nach Beispiel 10 hergestellten Methylesters 0,20 g der Titelverbindung als farbloses Öl.
IR: 3600, 3410 (breit), 2940, 2855, 1710, 1605, 976/cm.

**Beispiel 24**

**(13E)-(9S,11R,15S,16RS)-9-Brom-11,15-dihydroxy-16,19-dimethyl-13,18-prostadiensäure**

In Analogie zu Beispiel 14 erhält man aus 0,23 g des nach Beispiel 11 hergestellten Methylesters 0,19 g der Titelverbindung als farbloses Öl.
IR: 3600, 3410 (breit), 2938, 2854, 1710, 1605, 976/cm.

**Beispiel 25**

**(5Z,13E)-(9R,11R,15R)-9-Brom-11,15-dihydroxy-16,16,19-trimethyl-5,13,18-prostatriensäure**

In Analogie zu Beispiel 14 erhält man aus 0,2 g des nach Beispiel 12 hergestellten Methylesters 0,17 g der Titelverbindung als farbloses Öl.
IR: 3600, 3410 (breit), 2945, 2852, 1708, 1603, 976/cm.

**Beispiel 26**

**(5Z,13E)-(9S,11R,15R)-9-Brom-11,15-dihydroxy-16,16,19-trimethyl-5,13,18-prostatriensäure**

In Analogie zu Beispiel 14 erhält man aus 0,2 g des nach Beispiel 13 hergestellten Methylesters 0,17 g der Titelverbindung als farbloses Öl.
IR: 3610, 3410 (breit), 2948, 2850, 1710, 1604, 976/cm.

**Beispiel 27**

**(13E)-(9R,11R,15R)-9-Brom-1,11,15-trihydroxy-16-phenoxy-17,18,19,20-tetranor-13-prosten**

Zu einer Lösung von 250 mg (13E)-(9R,11R,15R)-9-Brom-11,15-dihydroxy-16-phenoxy-17,18,19,20-tetranor-13-prostensäure-methylester (hergestellt nach Beispiel 3) in 10 ml Tetrahydrofuran fügt man bei 0 °C unter Rühren portionsweise 300 mg Lithiumaluminiumhydrid und rührt anschließend 30 Minuten bei 0°C. Man zerstört den Reagenzüberschuß bei 0 °C durch tropfenweise Zugabe von Essigester, fügt 1 ml Wasser und 50 ml Äther zu und rührt intensiv 3 Stunden bei 25 °. Man filtriert, wäscht den Rückstand mit Äther, trocknet die ätherische Lösung über Magnesiumsulfat und dampft im Vakuum ein. Nach Chromatographie des Rückstands an Kieselgel mit Essigester/Hexan (4:1) erhält man 205 mg der Titelverbindung als farbloses Öl.
IR: 3600, 3425 (breit), 2950, 2858, 1600, 1588, 1485, 978/cm.

**Beispiel 28**

**(13E)-(9S,11R,15R)-9-Brom-1,11,15-trihydroxy-16-phenoxy-17,18,19,20-tetranor-13-prosten**

In Analogie zu Beispiel 27 erhält man aus 200 mg (13E)-(9S,11R,15R)-9-Brom-11,15-dihydroxy-16-phenoxy-17,18, 19,20-tetranor-13-prostensäuremethylester (hergestellt nach Beispiel 4) 150 mg der Titelverbindung als farbloses Öl.
IR: 3600, 3420 (breit), 2948, 2858, 1600, 1588, 1480, 976/cm.

**Beispiel 29**

**(13E)-(9R,11R,15R)-9-Brom-11,15-dihydroxy-16-phenoxy— 17,18,19,20-tetranor-13-prostensäure-methylsulfonamid**

Zu einer Lösung von 200 mg (13E)-(9R,11R,15R)-9-Brom-11,15-dihydroxv-16-phenoxy-17,18,19,20-tetranor-13-prostensäure (hergestellt nach Beispiel 16) in 5 ml Dimethylformamid gibt man bei -10 °C 85 mg Chlorameisensäureisobutylester und 66 mg Triäthylamin. Nach 60 Minuten gibt man 280 mg des Natriumsalzes von Methylsulfonamid (hergestellt aus Methylsulfonamid und Natriummethylat) und 2 mlHexamethylphosphorsäuretriamid zu und rührt 5 Stunden bei +10 °C. Anschließend verdünnt man mit Citratpuffer (pH 4), extrahiert mit Essigester, wäscht den Extrakt mit Salz, trocknet über Magnesiumsulfat und dampft im Vakuum ein. Nach Chromatographie des Rückstands an Kieselgel mit Methylenchlorid erhält man 130 mg der Titelverbindung als Öl.
IR: 3600, 3410, 2954, 2860, 1718, 1600, 1588, 976/cm.

**Beispiel 30**

**(13E)-(9S,11R,15R)-9-Brom-11,15-dihydroxy-16-phenoxy— 17,18,19,20-tetranor-13-prostensäure-methylsulfonamid**

In Analogie zu Beispiel 29 erhält man aus 100 mg (13E)-(9S,11R,15R)-9-Brom-11,15-dihydroxy-l6;phenoxy-17,18, 19,20-tetranor-13-prostensäure (hergestellt nach Beispiel 17) 60 mg der Titelverbindung als farbloses Öl.
IR: 3600, 3405, 2956, 2860, 1718, 1601, 1588, 976/cm.

**Beispiel 31**

**(13E)-(9R,11R,15R)-9-Brom-11,15-dihydroxy-16-phenoxy— 17,18,19 20-tetranor-13-prostensäureamid**

Zu einer Lösung von 200 mg (138)-(9R,11R,15R)-9-Brom-11,15-dihydroxy-16-phenoxy-17,18,19,20-tetranor-13-prostensäure in 5 ml Tetrahydrofuran gibt man bei -10 °C 85 mg Chlorameisensäureisobutylester und 66 mg Triäthylamin. Nach 1 Stunde leitet man bei -10 °C 15 Minuten lang Ammoniakgas ein und läßt 2 Stunden bei -5 °C stehen. Anschließend verdünnt man mit Wasser, extrahiert mehrmals mit Methylenchlorid, wäscht die vereinigten Extrakte mit Sole, trocknet über Magnesiumsulfat und dampft im Vakuum ein. Zur Reinigung wird an Kieselgel mit Methylenchlorid chromatographiert. Man erhält 140 mg der Titelverbindung als Öl.
IR: 3600, 3450, 2958, 2840, 1668, 1608, 1590, 976/cm.

**Beispiel 32**

**(13E)-(9R,11R,15R)-9-Brom-11,15-dihydroxy-16-phenoxy— 17,18,19,20-tetranor-prostensäure-tris-(hydroxymethyl)— aminomethan-Salz**

Zu einer Lösung von 200 mg (13E)-(9R,11R,15R)-9-Brom-11,15-dihydroxy-16-phenoxy-17,18,19,20-tetranor-prostensäure (hergestellt nach Beispiel 16) in 35 ml Acetonitril gibt man bei 65 °C eine Lösung von 55 mg Tris(hydroxymethyl)-aminomethan in 0,18 ml Wasser. Unter Rühren läßt man auf 20 °C abkühlen, dekantiert vom Lösungsmittel und trocknet den Rückstand im Vakuum. Man erhält 175 mg der Titelverbindung als zähes Öl.

**Patentansprüche**

1. 9-Brom-Prostanderivate der allgemeinen Formel I

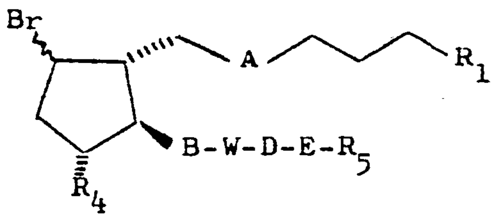

(I),

worin das 9-Bromatom α- oder β-ständig sein kann,
$R_1$ den Rest $-CH_2OH$ oder

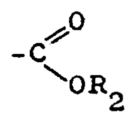

mit $R_2$ in der Bedeutung eines Wasserstoffatoms; einer $C_{1-10}$-Alkylgruppe; einer $C_{1-10}$-Alkylgruppe, die substituiert ist durch einen Fluor-, Chlor-, Brom-, Phenyl-, Dimethylamino-, Diäthylamino-, Methoxy-, Äthoxy- oder einen durch den Chlor-, Brom-, Fluor-, Phenyl-, Methoxy- oder Äthoxy-Rest substituierten Phenyl-Rest; einer $C_{3-10}$-Cycloalkyl-gruppe; einer durch einen $C_{1-4}$-Alkyl-Rest substituierten $C_{3-10}$ -Cycloalkylgruppe; einer Phenyl- oder Naphthylgruppe; einer Phenyl- oder Naphthylgruppe, die substituiert ist durch einen Phenyl-, Chlormethyl-, Fluormethyl-, Trifluormethyl-, Carboxyl-, Hydroxy-Rest, 1-3 Halogen-Atome, 1-3 $C_1$-$C_4$-Alkylgruppen oder einer $C_{1-4}$-Alkoxygruppe; eines 5-oder 6-gliedrigen Heterocyclus mit wenigstens einem Stickstoff-, Sauerstoff- oder Schwefel-Atom;
oder $R_1$ den Rest

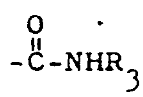

mit
$R_3$ in der Bedeutung eines Säurerestes oder des Restes $R_2$,
A eine $-CH_2-CH_2-$ oder cis-$CH=CH$-Gruppe,
B eine $-CH_2-CH_2-$, oder trans-$CH=CH-$ oder eine $-C\equiv C$-Gruppe,
W eine freie oder funktionell abgewandelte Hydroxymethylengruppe oder eine freie oder funktionell abgewandelte

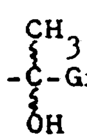-Gruppe,

wobei die OH-Gruppe α- oder β-ständig sein kann,
D und E gemeinsam eine direkte Bindung oder
D eine geradkettige, verzweigtkettige oder ringförmige Alkylengruppe mit bis zu 10 C-Atomen, die gegebenenfalls durch Fluoratome substituiert sein kann, und gegebenenfalls eine Doppelbindung in 2,3-oder 4-Stellung enthält,
E ein Sauerstoffatom oder Schwefelatom oder eine direkte Bindung oder eine $-C\equiv C$-Gruppe oder eine $-CR_6=CR_7$-Gruppe darstellt, wobei $R_6$ und $R_7$ sich unterscheiden und ein Wasserstoffatom oder ein Chloratom oder eine $C_1$-$C_4$-Alkyl-gruppe bedeuten,
$R_4$ eine freie oder funktionell abgewandelte Hydroxygruppe,
$R_5$ eine Alkyl- oder Alkenylgruppe mit bis zu 10 C-Atomen; eine Alkinylgruppe mit bis zu 6 C-Atomen; eine Alkinylgruppe mit bis zu 6 C-Atomen, die in 1-Stellung substituiert ist durch Fluor oder einen $C_{1-4}$-Alkylrest; eine $C_{3-10}$-Cycloalkylgruppe; eine durch einen $C_{1-4}$-Alkyl-Rest substituierte $C_{3-10}$-Cycloalkylgruppe; eine Phenyl- oder Naphthylgruppe; eine Phenyl- oder Naphthylgruppe, die substituiert ist durch einen Phenyl-, Chlormethyl-, Fluormethyl-, Trifluormethyl-, Carboxyl-, Hydroxy-Rest, 1-3 Halogenatome, 1-3 $C_{1-4}$-Alkylgruppen oder eine $C_{1-4}$-Alkoxygruppe; einen 5- oder 6-gliedrigen Heterocyclus mit wenigstens einem Stickstoff-, Sauerstoff- oder Schwefelatom; und falls $R_2$ die Bedeutung eines Wasserstoffatoms hat, deren Salze mit physiologisch verträglichen Basen bedeuten, mit der Maßgabe, daß die Gruppierung D-E-$R_5$ nicht den Rest 1,1-Dimethylpentyl

darstellt, wenn A für cis-CH=CH, B für trans-CH=CH-, W für CHOH, $R_4$ für OH, $R_1$ für $-COOCH_3$ stehen und der 9-Brom-Substituent β-ständig ist.

2. Verfahren zur Herstellung von 9-Brom-prostanderivaten der allgemeinen Formel I, dadurch gekennzeichnet, daß man eine Verbindung der allgemeinen Formel II

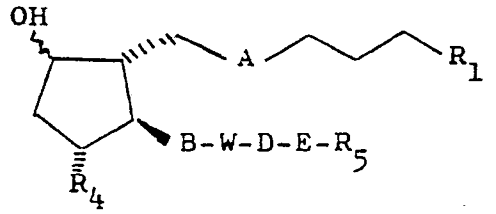

(II)

worin die OH-Gruppe α- oder β-ständig sein kann, $R_1$ die Reste

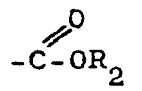 oder 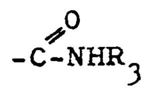

(mit $R_2$ außer Wasserstoff und $R_3$ in den bereits angegebenen Bedeutungen) darstellt und A, B, D, E und $R_5$ die oben angegebenen Bedeutungen haben und freie OH-Gruppen in $R_4$ und W geschützt sind, mit dem Reagenz Tetrabrommethan/Triphenylphosphin bromiert und gegebenenfalls anschließend in beliebiger Reihenfolge geschützte Hydroxygruppen freisetzt und/oder freie Hydroxygruppen verestert, veräthert und/oder Doppelbindungen hydriert und/oder eine veresterte Carboxylgruppe ($R_1$ =

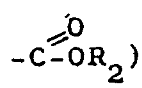)

verseift und/oder eine freie Carboxylgruppe ($R_2$ = H) in ein Amid ($R_1$ =

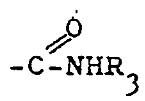)

überführt und/oder eine freie oder veresterte Carboxylgruppe ($R_1$ =

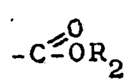)

reduziert.

3. Arzneimittel, bestehend aus einer oder mehreren Verbindungen gemäß Anspruch 1 und üblichen Hilfs- und Trägerstoffen.

4. (5Z,13E)-(9R,11R,15R)-9-Brom-11,15-dihydroxy-16-phenoxy-17,18,19,20-tetranor-5,13-prostadiensäuremethylester.

5. (5Z,13E)-(9S,11R,15R)-9-Brom-11,15-dihydroxy-16-phenoxy-17,18,19,20-tetranor-5,13-prostadiensäuremethylester.

6. (13E)-(9R,11R,15R)-9-Brom-11,15-dihydroxy-16-phenoxy-17,18,19,20-tetranor-13-prostensäure-methylester.

7. (13E)-(9S,11R,15R)-9-Brom-11,15-dihydroxy-16-phenoxy-17,18,19,20-tetranor-13-prostensäure-methylester.

8. (5Z,13E)-(9S,11R,15R)-9-Brom-11,15-dihydroxy-16,16-dimethyl-5,13-prostadiensäuremethylester.

9. (13E)-(9R,11R,15S,16RS)-9-Brom-11,15-dihydroxy-16,19-dimethyl-13,18-prostadiensäuremethylester.

10. (13E)-(9S,11R,15S,16RS)-9-Brom-11,15-dihydroxy-16,19-dimethyl-13,18-prostadiensäuremethylester.

11. (5Z,13E)-(9R,11R,15R)-9-Brom-11,15-dihydroxy-16,16,19-trimethyl-5,13,18-prostatriensäuremethylester.
12. (5Z,13E)-(9S,11R,15R)-9-Brom-11,15-dihydroxy-16,16,19-trimethyl-5,13,18-prostatriensäuremethylester.
13. (5Z,13E)-(9R,11R,15R)-9-Brom-11,15-dihydroxy-16-phenoxy-17,18,19,20-tetranor-5,13-prostadiensäure.
14. (5Z,13E)-(9S,11R,15R)-9-Brom-11,15-dihydroxy-16-phenoxy-17,18,19,20-tetranor-5,13-prostadiensäure.
15. (13E)-(9R,11R,15R)-9-Brom-11,15-dihydroxy-16-phenoxy-17,18,19,20-tetranor-13-prostensäure.
16. (13E)-(9S,11R,15R)-9-Brom-11,15-dihydroxy-16-phenoxy-17,18,19,20-tetranor-13-prostensäure.
17. (5Z,13E)-(9R,11R,15R)-9-Brom-11,15-dihydroxy-16,16-dimethyl-5,13-prostadiensäure.
18. (5Z,13E)-(9S,11R,15R)-9-Brom-11,15-dihydroxy-16,16-dimethyl-5,13-prostadiensäure.
19. (13E)-(9R,11R,15S,16RS)-9-Brom-11,15-dihydroxy-16,19-dimethyl-13,18-prostadiensäure.
20. (13E)-(9S,11R,15S,16RS)-9-Brom-11,15-dihydroxy-16,19-dimethyl-13,18-prostadiensäure.
21. (5Z,13E)-(9R,11R,15R)-9-Brom-11,15-dihydroxy-16,16,19-trimethyl-5,13,18-prostatriensäure.
22. (5Z 13E)-(9S IIR 15R)-9-Brom-11,15-dihydroxy-16,16,19-trimethyl-5,13,18-prostatriensäure.
23. (13E)-(9R,11R,15R)-9-Brom-1,11,15-trihydroxy-16-phenoxy-17,18,19,20-tetranor-13-prosten.
24. (138)-(9S,11R,15R)-9-Brom-1,11,15-trihydroxy-16-phenoxy-17,18,19,20-tetranor-13-prosten.
25. (13E)-(9R,11R,15R)-9-Brom-11,15-dihydroxy-16-phenoxy-17,18,19,20-tetranor-13-prostensäure-methylsulfonamid.
26. (13E)-(9S,11R,15R)-9-Brom-11,15-dihydroxy-16-phenoxy-17,18,19,20-tetranor-13-prostensäure-methylsulfonamid.
27. (13E)-(9R,11R,15R)-9-Brom-11,15-dihydroxy-16-phenoxy-17,18,19,20-tetranor-13-prostensäureamid.
28. (13E)-(9R,11R,15R)-9-Brom-11,15-dihydroxy-16-phenoxy-17,18,19,20-tetranor-prostensäure-tris-(hydroxymethyl)— aminomethan-Salz.

## Claims

1. 9-Bromoprostane derivatives of the general formula I

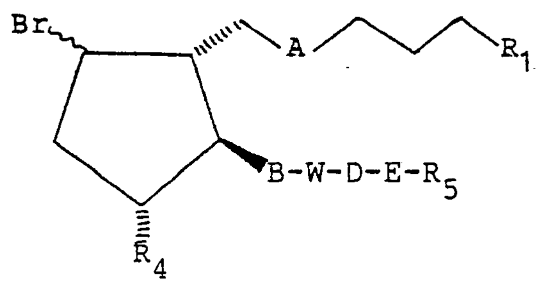

(I),

in which the 9-bromine atom may be in the α- or β-configuration,
$R_1$ represents the radical $-CH_2OH$ or

$R_2$
representing a hydrogen atom; a $C_{1-10}$-alkyl group; a $C_{1-10}$-alkyl group that is substituted by a fluorine, chlorine, bromine, phenyl, dimethylamino, diethylamino, methoxy or ethoxy radical or by a phenyl radical that is substituted by a chlorine, bromine, fluorine, phenyl, methoxy or ethoxy radical; a $C_{3-10}$-cycloalkyl group; a $C_{3-10}$-cycloalkyl group substituted by a $C_{1-4}$-alkyl radical; a phenyl or naphthyl group; a phenyl or naphthyl group that is substituted by a phenyl, chloromethyl, fluoromethyl, trifluoromethyl, carboxy or hydroxy radical, by 1-3 halogen atoms, 1-3 $C_1$-$C_4$-alkyl groups or by a $C_{1-4}$-alkoxy group, or $R_2$ representing a 5- or 6-membered heterocycle having at least one nitrogen, oxygen or sulphur atom; or
$R_1$ represents the radical

FIG30/20

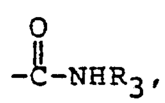

$R_3$ representing an acid radical or the radical $R_2$,
A represents a $-CH_2-CH_2-$ or cis-$CH=CH-$ group,
B represents a $-CH_2-CH_2-$, a trans-$CH=CH-$ or a $-C\equiv C-$ group,
W represents a free or functionally modified hydroxymethylene group or a free or functionally modified

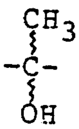

group in which the OH group may be in the α- or β-configuration,

D and E together represent a direct bond, or

D represents a straight-chained, branched or cyclic alkylene group having up to 10 carbon atoms, which may optionally be substituted by fluorine atoms and optionally contains a double bond in the 2-, 3- or 4-position,

E represents an oxygen or sulphur atom or a direct bond or a -C≡C- group or a $-CR_6=CR_7-$ group in which $R_6$ and $R_7$ are different from one another and each represents a hydrogen atom or a chlorine atom or a $C_1$-$C_4$-alkyl group,

$R_4$ represents a free or functionally modified hydroxy group,

$R_5$ represents an alkyl or alkenyl group having up to 10 carbon atoms; an alkynyl group having up to 6 carbon atoms; an alkynyl group having up to 6 carbon atoms that is substituted in the 1-position by fluorine or a $C_{1-4}$-alkyl radical; a $C_{3-10}$-cycloalkyl group; a $C_{3-10}$-cycloalkyl group substituted by a $C_{1-4}$-alkyl radical; a phenyl or naphthyl group; a phenyl or naphthyl group that is substituted by a phenyl, chloromethyl, fluoromethyl, trifluoromethyl, carboxy or hydroxy radical, by 1-3 halogen atoms, 1-3 $C_{1-4}$-alkyl groups or by a $C_{1-4}$-alkoxy group, or a 5- or 6-membered heterocycle having at least one nitrogen, oxygen or sulphur atom;

and if $R_2$ represents a hydrogen atom, the salts thereof with physiologically tolerable bases,

with the proviso that the grouping $D$-$E$-$R_5$ does not represent the radical 1,1-dimethylpentyl when A represents cis-CH=CH-, B represents trans-CH=CH-, W represents CHOH, $R_4$ represents OH, $R_1$ represents $-COOCH_3$ and the 9-bromo substituent is in the β-configuration.

2. Process for the manufacture of 9-bromoprostane derivatives of the general formula I, characterised in that a compound of the general formula II

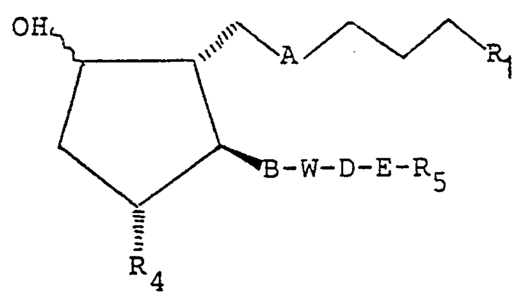

(II)

in which the OH group may be in the α- or β-configura-tion, $R_1$ represents the radical

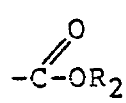 or 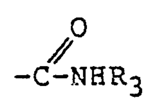

($R_2$ having the meanings already given with the exception of hydrogen and $R_3$ having the meanings already given) and A, B, D, E and $R_5$ have the meanings given above and free OH groups in $R_4$ and W are protected,

is brominated with the reagent tetrabromomethane/triphenylphosphine, and optionally then, in any sequence, protected hydroxy groups are freed and/or free hydroxy groups are esterified or etherified and/or double bonds are hydrogenated and/or an esterified carboxy group ($R_1$ =

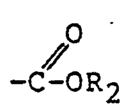)

is hydrolysed and/or a free carboxy group ($R_2$ = H) is converted into an amide ($R_1$ =

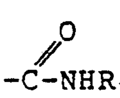)

and/or a free or an esterified carboxy group ($R_1$ =

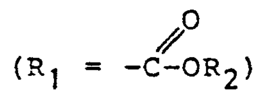

is reduced.

3. Medicaments consisting of one or more compounds according to claim 1 and customary adjuncts and carriers.

4. (5Z,13E)-(9R,11R,15R)-9-Bromo-11,15-dihydroxy-16-phenoxy-17,18,19,20-tetranor-5,13-prostadienoic acid methyl ester.

5. (5Z,13E)-(9S,11R,15R)-9-Bromo-11,15-dihydroxy-16-phenoxy-17,18,19,20-tetranor-5,13-prostadienoic acid methyl ester.

6. (13E)-(9R,11R,15R)-9-Bromo-11,15-dihydroxy-16-phenoxy-17,18,19,20-tetranor-13-prostenoic acid methyl ester.

7. (13E)-(9S,11R,15R)-9-Bromo-11,15-dihydroxy-16-phenoxy-17,18,19,20-tetranor-13-prostenoic acid methyl ester.

8. (5Z,13E)-(9S,11R,15R)-9-Bromo-11,15-dihydroxy-16,16-dimethyl-5,13-prostadienoic acid methyl ester.

9. (13E)-(9R,11R,15S,16RS)-9-Bromo-11,15-dihydroxy-16,19-dimethyl-13,18-prostadienoic acid methyl ester.

10. (13E)-(9S,11R,15S,16RS)-9-Bromo-11,15-dihydroxy-16,19-dimethyl-13,18-prostadienoic acid methyl ester.

11. (5Z,13E)-(9R,11R,15R)-9-Bromo-11,15-dihydroxy-16,16,19-trimethyl-5,13,1β-prostatrienoic acid methyl ester.

12. (5Z,13E)-(9S,11R,15R)-9-Bromo-11,15-dihydroxy-16,16,19-trimethyl-5,13,18-prostatrienoic acid methyl ester.

13. (5Z,13E)-(9R,11R,15R)-9-Bromo-11,15-dihydroxy-16-phenoxy-17,18,19,20-tetranor-5,13-prostadienoic acid.

14. (5Z,13E)-(9S,11R,15R)-9-Bromo-11,15-dihydroxy-16-phenoxy-17,18,19,20-tetranor-5,13-prostadienoic acid.

15. (13E)-(9R,11R,15R)-9-Bromo-11,15-dihydroxy-16-phenoxy-17,18,19,20-tetranor-13-prostenoic acid.

16. (13E)-(9S,11R,15R)-9-Bromo-11,15-dihydroxy-16-phenoxy-17,18,19,20-tetranor-13-prostenoic acid.

17. (5Z,13E)-(9R,11R,15R)-9-Bromo-11,15-dihydroxy-16,16-dimethyl-5,13-prostadienoic acid.

18. (5Z,13E)-(9S,11R,15R)-9-Bromo-11,15-dihydroxy-16,16-dimethyl-5,13-prostadienoic acid.

19. (13E)-(9R 11R, 15S, 16RS)-9-Bromo-11,15-dihydroxy-16,19-dimethyl-13,18-prostadienoic acid.

20. (13E)-(9S,11R,15S,16RS)-9-Bromo-11,15-dihydroxy-16,19-dimethyl-13,18-prostadienoic acid.

21. (5Z,13E)-(9R,11R,15R)-9-Bromo-11,15-dihydroxy-16,16,19-trimethyl-5,13,18-prostatrienoic acid.

22. (5Z,13E)-(9S,11R,15R)-9-Bromo-11,15-dihydroxy-16,16,19-trimethyl-5,13,18-prostatrienoic acid.

23. (13E)-(9R,11R,15R)-9-Bromo-1,11,15-trihydroxy-16-phenoxy-17,18,19,20-tetranor-13-prostene.

24. (13E)-(9S,11R,15R)-9-Bromo-1,11,15-trihydroxy-16-phenoxy-17,18,19,20-tetranor-13-prostene.

25. (13E)-(9R,11R,15R)-9-Bromo-11,15-dihydroxy-16-phenoxy-17,18,19,20-tetranor-13-prostenoic acid methylsulphonamide.

26. (13E)-(9S,11R,15R)-9-Bromo-11,15-dihydroxy-16-phenoxy-17,18,19,20-tetranor-13-prostenoic acid methylsulphonamide.

27. (13E)-(9R,11R,15R)-9-Bromo-11,15-dihydroxy-16-phenoxy-17,18,19,20-tetranor-13-prostenoic acid amide.

28. (13E)-(9R, 11R, 15R)-9-Bromo-11,15-dihydroxy-16-phenoxy-17,18,19,20-tetranor-13-prostenoic acid tris(hydroxymethyl)-aminomethane salt.

## Revendications

1. Dérivés du bromo-9-prostane, de formule générale I:

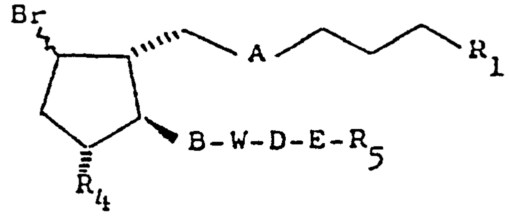

(I),

dans laquelle l'atome de brome en position 9 peut être en α ou β,

$R_1$ représente le reste $-CH_2OH$ ou bien

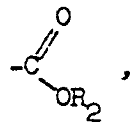 ,

où $R_2$ représente un atome d'hydrogène; un groupe alkyle en $C_1$ à $C_{10}$; un groupe alkyle en $C_1$ à $C_{10}$, qui est substitué par un reste fluoro, chloro, bromo, phényle, diméthylamino, diéthylamio, méthoxy ou éthoxy ou bien un reste phényle substitué par un reste chloro, bromo, fluoro, phényle, méthoxy ou éthoxy; un groupe cycloalkyle en $C_3$ à $C_{10}$; un groupe cycloalkyle en $C_3$ à $C_{10}$, qui est substitué par un reste alkyle en $C_1$ à $C_4$; un groupe phényle ou naphtyle éventuellement substitué par un reste phényle, chlorométhyle, fluorométhyle, trifluorométhyle, carboxyle, hydroxyle, 1 à 3 atomes d'halogène, 1 à 3 groupes alkyles en $C_1$ à $C_4$ ou un groupe alcoxy en $C_1$ à $C_4$; un hétérocycle à 5 ou 6 chaînons comportant au moins un atome d'azote, d'oxygène ou desoufre;

ou bien $R_1$ représente le reste

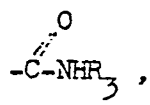 ,

dans lequel $R_3$ a le sens d'un reste acide ou du reste $R_2$,

A représente un groupe $-CH_2-CH_2-$ ou $-CH=CH-$cis,

B représente un groupe $-CH_2-CH_2-$, ou $-CH=CH-$trans ou un groupe $-C\equiv C$,

W représente un groupe hydroxyméthylène libre ou fonctionnellement modifié, ou un groupe:

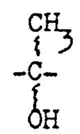

libre ou fonctionnellement modifié, dans lequel le groupe OH peut être en α ou en β,

D et E ensemble forment une liaison directe, ou bien

D représente un groupe alkylène linéaire, ramifié ou cyclique ayant jusqu'à 10 atomes de carbone, qui peut éventuellement être substitué par des atomes de fluor et qui a éventuellement une double liaison en position 2, 3 ou 4 du reste alkylène,

E représente un atome d'oxygène ou un atome de soufre ou une liaison directe ou un groupe $-C\equiv C-$ ou un groupe $-CR_6=CR_7-$, où $R_6$ et $R_7$ sont différents

l'un de l'autre et représentent un atome d'hydrogène ou un atome de chlore ou un groupe alkyle en $C_1$ à $C_4$,

$R_4$ est un groupe hydroxyle libre ou fonctionnellement modifié,

$R_5$ représente un groupe alkyle ou alcényle ayant jusqu'à 10 atomes de carbone; un groupe alcynyle ayant jusqu'à 6 atomes de carbone; un reste alcynyle ayant jusqu'à 6 atomes de carbone, et est substitué en position 1 par du fluor ou par un reste alkyle en $C_1$ à $C_4$; un groupe cycloalkyle en $C_3$ à $C_{10}$; un groupe cycloalkyle en $C_3$ à $C_{10}$ et qui est substitué par un reste alkyle en $C_1$ à $C_4$; un groupe phényle ou naphtyle éventuellement substitué par un reste phényle, chlorométhyle, fluorométhyle, trifluorométhyle, carboxyle, hydroxyle, 1 à 3 atomes d'halogène, 1 à 3 groupes alkyles en $C_1$ à $C_4$ ou un groupe alcoxy en $C_1$ à $C_4$; un hétérocycle à 5 ou 6 chaînons comportant au moins un atome d'azote, d'oxygène ou de soufre; et, si $R_2$ représente un atome d'hydrogène, ses sels avec des bases physiologiquement acceptables, à la condition que le groupement $D-E-R_5$ ne représente pas le reste diméthyl-1,1 pentyle lorsque A représente un groupe $-CH=CH-$cis, B représente un groupe $-CH=CH-$trans, W représente CHOH, $R_4$ représente OH, $R_1$ représente $-COOCH_3$ et le substituant brome en position 9 est en β.

2. Procédé pour préparer des dérivés du bromo-9 prostane de formule générale I, caractérisé en ce qu'on brome, à l'aide du réactif tétrabromo méthane/triphénylphosphine, un composé de formule générale II:

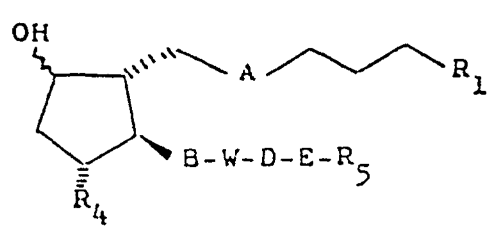 (II)

(dans laquelle le groupe OH peut se trouver en position α ou β, $R_1$ représente les restes

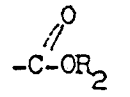 ou bien 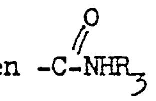

($R_2$ ayant un sens autre que hydrogène et $R_3$ ayant les sens déjà indiqués) et A, B, D, E et $R_5$ ont les sens indiqués ci-dessus, et les groupes OH libres en $R_4$ et W sont protégés), et, éventuellement ensuite, on effectue dans un ordre quelconque une libération des groupes hydroxyles protégés et/ou une estérification ou éthérification des groupes hydroxyles libres et/ou une hydrogénation des doubles liaisons et/ou une sapoüfication de groupes carboxyles estérifié ($R_1$ =

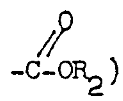

et/ou la transformation d'un groupe carboxyle libre ($R_2$ = H) en un amide ($R_1$ =

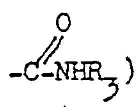

et/ou la réduction d'un groupe carboxyle libre ou estérifié ($R_1$ =

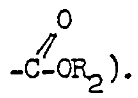

3. Médicament consistant en un ou plusieurs composés selon la revendication 1 et en les adjuvants et supports usuels.
4. (Z5,E13)-(R9,R11,R15)-bromo-9 dihydroxy-11,15 phénoxy-16 tétra-nor-17, 18, 19,20 prostadiène-5, 13 oate de méthyle.
5. (Z5,E13)-(59,R11,R15)-bromo-9 dihydroxy-11,15 phénoxy- 16 tétra-nor-17,18,19,20 prostadiène-5,13 oate de méthyle.
6. (E13)- (R9,R11,R15)-bromo-9 dihydroxy-11,15 phénoxy-16 tétranor-17,18,19,20 prostène-13 oate de méthyle.
7. (E13)- (S9,R11,R15)-bromo-9 dihydroxy-11,15 phénoxy-16 tétranor-17,18,19,20 prostène-13 oate de méthyle.
8. (Z5,E13)- (S9,R11,R15)-bromo-9 dihydroxy-11,15 diméthyl-16,16 prostadène-5,13 oate de méthyle.
9. (E13)- (R9,R11,S15,RS16)-bromo-9 dihydroxy-11,15 diméthyl-16, 19 prostadiène-13,18 oate de méthyle.
10. (E13)- (S9,R11,S15,RS16)-bromo-9 dihydroxy-11, 15 diméthyl-16, 19 prostadiène-13,18 oate de méthyle.
11. (Z5,E13)- (R9,R11,R15)-bromo-9 dihydroxy-11, 15 triméthyl-16,16,19 prostatriène-5,13,18 oate de méthyle.
12, (ZS, E13)-(S9, R1,R15)-bromo- dihydroxy-11,15 triméthyl-16,16,19 prostatriène-5,1$_3$,18 oate de méthyle.
13, Acide (Z5,E13)-(R9,R11,R15)-bromo-9 dihydroxy-11,15 phénoxy-16 tétranor-17,18,19,20 prostadiène-5,13 oïque.
14. Acide (Z5, E13)-(S9,R11,R15)-bromo-9 dihydroxy-11,15 phénoxy-16 tétranor-17,18,19,20 prostadiène-5,13 oïque.
15. Acide (E13)-(R9,R11, R15)-bromo-9 dihydroxy-11,15 phénoxy-16 tétra-nor-17,18,19,20 prostène-13 oïque.
16. Acide (E13)-(S9,R11,R15)-bromo-9 dihydroxy-11,15 phénoxy-16 tétra-nor-17,18,19,20 prostène-13 oïque.
17. Acide (Z5,E13)-(R9,R11,R15)- bromo-9 dihydroxy-11,15 diméthyl-16, 16 prostadiène-5,13 oïque.
18. Acide (Z5,E13)-(S9,R11,R15)-bromo-9 dihydroxy-11,15 diéthyl-16,16 prostadiène-5,13 oïque.
19. Acide (E13)-(R9,R11,S15,RS16)-bromo-9 dihydroxy-11,15 diméthyl-16, 19 prostadiène-13,15 oïque.
20. Acide (E13)-(S9,R11, S15, RS16) - bromo-9 dihydroxy-11,15 diméthyl- 16, 19 prostadiène-13,18 oïque.
21. Acide (Z5,E13)-(R9,R11,R15)-bromo-9 dihydroxy-11,15 triméthyl-16, 16,19 prostratriène-5,13,18 oïque.
22. Acide (Z5,E13)-(S9,R11,R15) - bromo-9 dihydroxy-11,15 triméthyl-16 16,19 prostatriène-5,13,18 oïque.
23. (E13)-(R9,R11,R15)-bromo-9 trihydroxy-1,11,15 phénoxy-16 tétranor-17,18,19,20 prostène-13.
24. (E13)-(S9,R11, R15)-bromo-9 trihydroxy-1,11,15 phénoxy-16 tétranor-17,18,19,20 prostène-13.
25. Méthylsulfenamide de l'acide (E13)-(R9,R11,R15)-bromo-9 dihydroxy-11,15 phénoxy-16 tétranor-17,18,19,20 prostène-13 oïque.
26. Méthylsulfonamide de l'acide (E13)-(S9,R11,R15)-bromo-9 dihydroxy-11,15 phénoxy-16 tétranor-17,18,19,20 prostène-13 oïque.

27. Amide de l'acide (E13)-(R9,R11,R15)-bromo-9 dihydroxy-11,15 phé-noxy-16 tétranor-17,18,19,20 prostène-13 oïque.

28. Sel de tris(hydroxyméthyl) aminométhane de l'acide (E13)-(R9,R11, R15)-bromo-9 dihydroxy-11,15 phénoxy-16 tétranor-17,18,19,20 prostène-13-oïque.